# EUROPEAN PATENT APPLICATION

(11) **EP 1 886 670 A1**
(43) Date of publication of application: **13.02.2008**
(21) Application number: 06254865.6
(22) Date of filing: 20.09.2006
(51) Int. Cl.: A61K 9/16, A61K 9/20, A61K 31/13

(54) **Pharmaceutical compositions of memantine**

(30) Priority: 05.07.2006 US 818823 P
(71) Applicant: Teva Pharmaceutical Industries Ltd, 49131 Petah Tiqva (IL)
(72) Inventor: Hrakovsky, Julia, Rosh Ha-Ayin 48057 (IL); Sebban, Hagit, Acco 24662 (IL)
(74) Representative: Russell, Tim

(57) **Abstract**

The invention is directed to easily dissolved, stable dose proportional pharmaceutical compositions, comprising granulated memantine and methods of preparing the same. In particular, the invention is directed to granulated memantine pharmaceutical compositions in the form of film coated tablets.

## Description

### Cross-reference to Related Applications

This application claims the benefit of U.S. provisional application No. 60/818,823, filed July 5, 2006, herein incorporated by reference.

### Field of the Invention

The invention is directed to easily dissolved, stable, dose proportional pharmaceutical compositions, comprising memantine, and methods of preparing the same. In particular, the invention is directed to granulated pharmaceutical compositions in the form of film coated tablets.

### Background of the Invention

Memantine is reportedly an orally active NMDA receptor antagonist. The hydrochloride salt of memantine is commercially available as NAMENDA®. The reported IUPAC name for memantine hydrochloride is 3,5-dimethyladamantan-1-amine hydrochloride, and memantine hydrochloride is also reportedly known by the chemical name 1-amino-3,5-dimethyladamantane hydrochloride. Memantine hydrochloride is understood to have the structural formula: 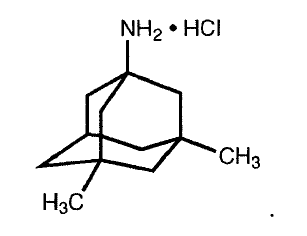 The molecular formula of memantine hydrochloride is C₁₂H₂₁N·HCl, and the molecular weight is 215.76.

Memantine HCl is currently marketed by Forest in the form of film coated tablets under the trade name NAMENDA®. In Europe, memantine hydrochloride is marketed by Merz (AXURA®) and Lundbeck (EBIXA®). NAMENDA® has been approved by the FDA for the treatment of moderate to severe Alzheimer's disease. The film coated NAMENDA® tablets reportedly contain memantine HCl and the following inactive ingredients: microcrystalline cellulose, lactose monohydrate, colloidal silicon dioxide, talc, magnesium stearate, hypromellose, triacetin, titanium dioxide, FD&C yellow #6, FD&C blue #2, and iron oxide black.

U.S. publication No. 2006/0002999 A1 ("the '999 publication"), the teachings of which are incorporated herein by reference in their entirety, reports pharmaceutical compositions of 1-aminocyclohexanes, such as memantine or neramexane, prepared by a direct compression method, which are released at a dissolution rate of at least about 80 percent in 60 minutes, and have a hardness between 3 and 40 Kp, i.e., between about 4.2 and 56 SCU (Strong Cobb Units).

Even though direct compression is a fairly simple process in theory, in practice it suffers from various disadvantages, e.g., requiring careful choice of ingredients to ensure fairly uniform particle sizes of all ingredients, so that segregation does not occur, and, thus, lack of homogeneity is a recurring problem in direct compression compositions. In other words, compositions prepared by direct compression processes suffer from a lack of uniformity. Direct compression compositions often require the use of flow enhancing ingredients and compression aids, as powders are more likely to suffer from poor flow, poor compressibility and sticking, and "capping," as disclosed in the '999 publication.

U.S. Patent Application Publication No. 2006/0198884 A1 ("the '884 publication"), the teachings of which are incorporated herein by reference in their entirety, reports pharmaceutical compositions of 1-aminocyclohexanes, such as memantine or neramexane. The terms used in this application are very unclear as to their exact meaning, but this application would seem to claim various dosage units of memantine comprising various amounts of memantine.

### Summary of the Invention

The present invention is directed to a granulated composition comprising memantine, wherein at about 95 percent of the memantine is released within about 60 minutes of being placed in 0.1 N HCl at 37°C. Preferably, about 95 percent of the memantine is released within about 45 minutes, more preferably within about 30 minutes, and most preferably within about 15 minutes of being placed in the 0.1 N HCl at 37°C.

Preferably, the memantine is in the form of memantine hydrochloride. More preferably, the memantine hydrochloride is present in an amount of about 2 to about 6 percent by weight of the composition.

In accordance with the invention, the granulated memantine composition preferably comprises memantine, and at least one pharmaceutically acceptable excipient. More preferably, the memantine is memantine hydrochloride. Preferably, the at least one pharmaceutically acceptable excipient is selected from the group consisting of diluents, binders, disintegrants, glidants, and lubricants. Preferred diluents include lactose monohydrate and microcrystalline cellulose. Preferably, the diluent is present in an amount of about 40 percent to about 95 percent by weight of the composition. Preferred binders include povidone. Preferably, the binder is present in an amount of about 0.5 percent to about 10 percent by weight of the composition. Preferred disintegrants include croscarmellose sodium. Preferably, the disintegrant is present in an amount of about 3 percent to about 10 percent by weight of the composition. Preferred glidants include colloidal silicon dioxide and talc. Preferably, the glidant is present in an amount of about 0.5 percent to about 3 percent by weight of the composition. Preferred lubricants include magnesium stearate. Preferably, the lubricant is present in an amount of about 0.5 percent to about 2 percent by weight of the composition.

In accordance with the invention, the granulated memantine or memantine hydrochloride composition is preferably in the form of a solid oral dosage form. Preferably, the solid oral dosage form is a tablet or a capsule or a sachet of granules.

In accordance with the invention, the granulated memantine composition is preferably in the form of a film-coated tablet comprising memantine, lactose monohydrate, povidone, microcrystalline cellulose, croscarmellose sodium, colloidal silicon dioxide, magnesium stearate, and a coating. Again, the memantine is preferably memantine hydrochloride.

The invention is also directed to a wet granulation process for preparing a memantine composition comprising granulating memantine and at least one pharmaceutically acceptable excipient, preferably a diluent, a binder, and/or a disintegrant, in a liquid to form a granulate, and drying and milling the granulate. The granulate is then preferably used to form a composition that, for example, may then be compressed into tablet cores or filled into capsules, such as by blending with one or more excipients, preferably a disintegrant, a glidant, and/or a lubricant. The granules can also be used "as-is," i.e., as the granules without any further processing.

The invention is also directed to a dry granulation process for preparing a memantine composition, comprising mixing memantine and at least one excipient, preferably a diluent, a binder, a disintegrant, and/or a lubricant, to form a mixture, compacting or slugging the mixture, and milling the mixture to form granules. The granules are then preferably used to form a composition that, for example, may then be compressed into tablet cores or filled into capsules, such as by blending with one or more excipients, preferably a disintegrant, a glidant, and/or a lubricant.

The invention is also directed to a granulated memantine composition prepared by the wet granulation process.

The invention is also directed to a granulated memantine composition prepared by the dry granulation process.

The invention is also directed to the use of a composition of the invention in therapy. As discussed above, memantine has been approved for use in treating Alzheimer's disease. Thus, the compositions of the invention may be approvable for treating Alzheimer's disease.

### Detailed Description of the Invention

The invention addresses the disadvantages of direct compression by providing memantine compositions prepared by granulation. The granulated memantine compositions of the invention have dissolution profiles comparable to or better than those reported for direct compression compositions, without the need to increase tablet hardness to avoid sticking to the punches or capping, as reported in the '999 publication. Moreover, the tablet hardness has no substantial effect on the dissolution rate. Granulation methods, particularly wet granulation, allow for good uniformity of the active substance within the composition and very good stability of the finished product during the stability testing under accelerated conditions (40°C and 75 percent relative humidity), as well as good flowability.

The invention provides a composition comprising granulated memantine. That is the invention provides a composition comprising memantine, prepared by granulation, where the granulation is preferably wet granulation. The granulated memantine composition of the invention is preferably an immediate-release composition, wherein about 95 percent of the memantine is released within about 60 minutes of being placed in 0.1 N HCl at 37°C in a basket at 100 rpm (USP apparatus I). Preferably, about 95 percent of the memantine is released within about 45 minutes, more preferably within about 30 minutes, and most preferably not less than about 85 percent within about 15 minutes of being placed in the 0.1 N HCl at 37°C in a basket at 100 rpm.

In wet granulation, the active ingredient is blended, preferably together with excipients in powder form, and then further mixed in the presence of a liquid, typically water, which causes the powders to clump into granules. The obtained granulate can be screened and/or milled, dried, and then screened and/or milled to the desired particle size. The granulate may then be combined with additional excipients and/or formulated into a solid dosage form.

In dry granulation, the active ingredient is blended, preferably together with excipients in powder form, then compacted into slugs or a sheet or ribbon, and then comminuted into compacted granules. The compacted granules may subsequently be formulated into a solid dosage form. Preferably, dry granulation is performed by either roller compaction or "slugging."

The composition of the invention may comprise memantine and at least one pharmaceutically acceptable excipient. Preferably, the composition comprises memantine, a diluent, a binder, a disintegrant, a glidant, a surfactant, a lubricant, and/or a coating. Preferably, the memantine is in the form of memantine hydrochloride, which, more preferably, is present in an amount of between about 2 to about 6 percent, preferably, about 3 to about 5 percent by weight of the composition.

Useful diluents include diluents commonly used in solid pharmaceutical compositions. For example, useful diluents include, but are not limited to, at least one of calcium phosphate (dibasic and/or tribasic), calcium sulfate, powdered cellulose, dextrates, dextrin, fructose, kaolin, lactitol, lactose, maltose, mannitol, microcrystalline cellulose, sorbitol, starch, and sucrose. Preferably, the diluent is at least one of lactose monohydrate or microcrystalline cellulose. More preferably, the diluent is present in an amount of about 40 percent to about 95 percent, preferably, about 50 to about 90 percent by weight of the composition.

Useful binders include binders commonly used in solid pharmaceutical compositions. For example, useful binders include, but are not limited to, at least one of acacia, alginic acid, carbomer, sodium carboxymethylcellulose, dextrin, ethylcellulose, gelatin, glucose, guar gum, hydroxypropyl cellulose, hydroxypropyl methylcellulose, maltose, methylcellulose, microcrystalline cellulose, polyethylene oxide, starch, or povidone. Although ethylcellulose may be used, the use of ethylcellulose may hamper dissolution, and is not the most preferred binder. Preferably, the binder is povidone. More preferably, the binder is present in an amount of about 0.5 percent to about 10 percent, preferably, about 1 to about 5 percent by weight of the composition.

Useful disintegrants include those disintegrants commonly used in solid pharmaceutical compositions. For example, useful disintegrants include, but are not limited to, at least one of croscarmellose sodium, crospovidone, microcrystalline cellulose, potassium polacrilin, sodium starch glycolate, Low-Substituted Hydroxypropyl Cellulose, and starch. Preferably, the disintegrant is croscarmellose sodium. More preferably, the disintegrant is present in an amount of about 3 percent to about 10 percent by weight of the composition.

Useful glidants include those glidants commonly used in solid pharmaceutical compositions. For example, useful glidants include, but are not limited to, at least one of colloidal silicon dioxide, magnesium trisilicate, powdered cellulose, starch, and talc. Preferably, the glidant is at least one of colloidal silicon dioxide and talc. More preferably, the glidant is present in an amount of about 0.5 percent to about 3 percent by weight of the composition.

Useful lubricants include those lubricants commonly used in solid pharmaceutical compositions. For example, useful lubricants include, but are not limited to, at least one of calcium stearate, glyceryl behenate, magnesium stearate, mineral oil, polyethylene glycol, sodium stearyl fumarate, stearic acid, talc, sodium lauryl sulfate, and zinc stearate. Preferably, the lubricant is magnesium stearate and/or sodium stearyl fumarate and/or talc. More preferably, the lubricant is present in an amount of about 0.5 percent to about 2 percent by weight of the composition.

The composition may be formulated into a solid dosage form for oral administration. Preferably, the solid dosage form is a tablet, a capsule, or granules. More preferably, the solid dosage form is a tablet. Most preferably, the solid-dosage form is a film-coated tablet.

In one particularly preferred embodiment, the composition is formulated into a film-coated tablet comprising memantine hydrochloride, lactose monohydrate, povidone, microcrystalline cellulose, croscarmellose sodium, colloidal silicon dioxide, magnesium stearate, and a coating. Preferably, the coating comprises Hypromellose, polyethylene glycol, Polysorbate, titanium dioxide and/or iron oxide. For example, the coating may be prepared from a commercially available powder mix such as OPADRY®. OPADRY®, available from Colorcon, which typically comprises Hypromellose, Polyethylene Glycol, Polysorbate, and Colorants, e.g. titanium dioxide and iron oxide.

The invention also provides a process for preparing a memantine composition by wet granulation. The process comprises granulating memantine and at least one pharmaceutically acceptable excipient, preferably a diluent, a binder, and/or a disintegrant, in a liquid to form a granulate. The granulate is then preferably dried and milled. The granulate is then preferably used to form a composition that, for example, may then be compressed into tablet cores or filled into capsules, such as by blending with one or more pharmaceutically acceptable excipients, preferably a disintegrant, a glidant, and/or a lubricant.

The invention also provides a process for preparing a memantine composition by dry granulation. The process comprises mixing memantine and at least one pharmaceutically acceptable excipient, preferably a diluent, a binder, a disintegrant, and/or a lubricant to form a mixture, compacting or slugging the mixture, and milling the slugs to form granules. The granules are then preferably used to form a composition that, for example, may then be compressed into tablet cores or filled into capsules, such as by blending with one or more pharmaceutically acceptable excipients, preferably a disintegrant, a glidant, and/or a lubricant.

The compositions of the invention may be administered to a mammal. Preferably, the mammal is a human, and the composition is administered as pharmaceutical compositions. Preferably, the pharmaceutical composition is administered to treat Alzheimer's disease, as per the approved indications for NAMENDA®, see prescribing information.

The amount of the memantine in a pharmaceutical composition is preferably an amount that provides a therapeutically effective amount of memantine. It will be appreciated that the amount of memantine used will differ according to the amount needed to effect the therapeutic response.

"Effective" or "therapeutically effective" amount of a drug or pharmacologically active agent means an amount of the drug or agent that is nontoxic and sufficient to provide the desired effect, e.g., treatment of moderate to severe dementia of the Alzheimer's type, if indeed memantine is useful for such treatment.

The amount of memantine compound administered and the dosing regimen used, will depend on the particular compound selected, the age and general condition of the subject being treated, the severity of the subject's condition, and the judgment of the prescribing physician.

As used herein, "treating," "treated," and "treatment" mean at least one of the following: reduction in severity and/or frequency of symptoms, elimination of symptoms and/or underlying cause, prevention of the occurrence of symptoms and/or their underlying cause, or improvement or remediation of damage. For example, the present method of "treating" Alzheimer's disease, as the term is used herein, thus encompasses both prevention of the disorder in a predisposed individual and treatment of the disorder in a clinically symptomatic individual. The inventors do not wish in any way to express an opinion as to the efficacy of memantine as a treatment for a particular disorder but rather, the inventors wish to provide compositions that should prove useful to physicians that wish to treat a conditions that is believed by the physician is responsive to memantine or has been reported as such in the medical literature.

Having thus described the invention with reference to particular preferred embodiments and illustrative examples, those in the art may appreciate modifications to the invention as described and illustrated that do not depart from the spirit and scope of the invention as disclosed in the specification. The following examples are set forth to aid in understanding the invention, but are not intended to, and should not be construed to limit its scope in any way. The examples do not include detailed descriptions of conventional methods. Such methods are well known to those of ordinary skill in the art, and are described in numerous publications.

### Examples

Unless otherwise specified, the methods of analysis used were as follows: Dissolution is carried out in a 6 vessel assembly, apparatus I (Baskets) in a medium of 900 ml of 0.2 percent NaCl in 0.1 N HCl at 37°C with rotation at 100 rpm. Sampling is at the specified times, followed by preparation by derivatization with dansyl chloride. Analysis by HPLC using a fluorescence detector with a C₁₈ column and a mobile phase of acetonitrile and water.

Assay is carried out using a GC apparatus equipped with a FID detector and an Rtx-5 amine crossbond 5 percent diphenyl-95 percent dimethyl polysiloxane column and helium carrier gas. Auto-sampler and gradient parameters are detailed in the method. The sample is dissolved in a solution of potassium carbonate with sodium chloride, followed by extraction with hexane containing xylene as an internal standard. The standards and samples are injected into the GC column for a run time of up to 35 minutes.

### Example 1: Memantine Composition Prepared by Wet Granulation

A mixture of memantine hydrochloride (5 mg/tablet), lactose monohydrate (70 mg/tablet), povidone (1 mg/tablet), and croscarmellose sodium (7 mg/tablet) **[Part I]** was granulated with purified water as a granulating agent to form a granulate. The granulate was dried, screened, and blended with a mixture of croscarmellose sodium (2 mg/tablet), microcrystalline cellulose (32.5 mg/tablet), and colloidal silicon dioxide (1 mg/tablet) **[Part II]** and magnesium stearate (1.5 mg/tablet) **[Part III]** to form a blend. The total blending time was 30 minutes. The blend was then compressed into tablet cores. The ingredients are summarized in Table 1.

**Table 1:**

| Ingredient | (mg/tablet) |
|---|---|
| **Part I:** | |
| Memantine HCl | 5 |
| Lactose Monohydrate | 70 |
| Povidone | 1 |
| Croscarmellose Sodium | 7 |
| | |

| **Part II** | |
|---|---|
| Croscarmellose Sodium | 2 |
| Microcrystalline Cellulose | 32.5 |
| Colloidal Silicon Dioxide | 1 |
| | |

| **Part III** | |
|---|---|
| Magnesium Stearate | 1.5 |
| | |
| Total | 120.0 |

### Example 2: Memantine Composition Prepared by Wet Granulation

A mixture of memantine hydrochloride (10 mg/tablet), lactose monohydrate (140 mg/tablet), povidone (2 mg/tablet), and croscarmellose sodium (14 mg/tablet) **[Part I]** was granulated with purified water as a granulating agent to form a granulate. The granulate was dried, milled, and blended with a mixture of croscarmellose sodium (4 mg/tablet), microcrystalline cellulose (65 mg/tablet), and colloidal silicon dioxide (2 mg/tablet) **[Part II]** and magnesium stearate (3 mg/tablet) **[Part III]** to form a blend. The total blending time was 30 minutes. The blend was then compressed into tablet cores. The ingredients are summarized in Table 2 below.

**Table 2:**

| Ingredient | (mg/tablet) |
|---|---|
| **Part I** | |
| Memantine HCl | 10 |
| Lactose Monohydrate | 140 |
| Povidone | 2 |
| Croscarmellose Sodium | 14 |
| | |

| **Part II** | |
|---|---|
| Croscarmellose Sodium | 4 |
| Microcrystalline Cellulose | 65 |
| Colloidal Silicon Dioxide | 2 |
| | |

| **Part III** | |
|---|---|
| Magnesium Stearate | 3 |
| | |
| Total | 240 |

### Example 3: Memantine Composition Prepared by Wet Granulation

A mixture of memantine hydrochloride (10 mg/tablet), lactose monohydrate (50 mg/tablet), microcrystalline cellulose (100 mg/tablet), and croscarmellose sodium (8 mg/tablet) **[Part I]** was granulated with purified water as a granulating agent to form a granulate. The granulate was dried, screened, and blended with a mixture of croscarmellose sodium (4 mg/tablet), lactose monohydrate (60 mg/tablet), and talc (5 mg/tablet) **[Part II**] and magnesium stearate (3 mg/tablet) **[Part III]** to form a blend. The total blending time was 30 minutes. The blend was then compressed into tablet cores. The ingredients are summarized in Table 3 below.

**Table 3:**

| Ingredient | (mg/tablet) |
|---|---|
| **Part I** | |
| Memantine HCl | 10 |
| Lactose Monohydrate | 50 |
| Microcrystalline Cellulose | 100 |
| Croscarmellose Sodium | 8 |
| | |

| **Part II** | |
|---|---|
| Croscarmellose Sodium | 4 |
| Lactose Monohydrate | 60 |
| Talc | 5 |
| | |

| **Part III** | |
|---|---|
| Magnesium Stearate | 3 |
| | |
| Total | 240 |

### Comparative Example 4: Memantine Composition Prepared by Direct Compression

Memantine hydrochloride (5 mg/tablet), lactose monohydrate (55 mg/tablet), microcrystalline cellulose (55 mg/tablet), colloidal silicon dioxide (1 mg/tablet), talc (2.5 mg/tablet), and magnesium stearate (1.5 mg/tablet) were sieved and blended together to form a blend. The total blending time was 30 minutes. The blend was then compressed into tablet cores, and coated. The ingredients are summarized in Table 4 below.

**Table 4:**

| Ingredient | (mg/tablet) |
|---|---|
| Memantine HCl | 5 |
| Lactose Monohydrate | 55 |
| Microcrystalline Cellulose | 55 |
| Colloidal Silicon Dioxide | 1 |
| Talc | 2.5 |
| Magnesium Stearate | 1.5 |
| OPADRY® | 3.6 |
| | |
| Total | 123.6 |

The pharmaceutical compositions prepared in Examples 1 and 2 were tested for assay and dissolution. The results are summarized in Table 5.

**Table 5**

| Sample | Amount of memantine HCl | Dissolution in 60 min (%) | Assay (%) |
|---|---|---|---|
| Example 1 | 5 mg/tablet | 102 | 98.5 |
| Example 2 (Hardness = 15 SCU) | 10 mg/tablet | 101 | 101.7 |

The rate of dissolution of the pharmaceutical composition prepared in Example 3 was tested using a U.S.P. Type I Apparatus (basket) with 900 ml of 0.1 N HCl at 37°C and a rotation speed of 100 rpm. The dissolution profile of the pharmaceutical composition prepared in Example 3 is summarized in Table 6.

**Table 6**

| Sample | % Dissolved | | | |
|---|---|---|---|---|
| | 15 min | 30 min | 45 min | 60 min |
| Example 3 (Hardness = 7 SCU) | 96 | 96 | 96 | 96 |

The pharmaceutical compositions prepared in Examples 1 (wet granulation) and 4 (direct compression) were tested for blend uniformity. The final blend was sampled with ten samples taken from different places in the storage container, and every sample was tested for assay. The results are summarized in Table 7, where "RSD" refers to the relative standard deviation.

**Table 7**

| Sample | Blend Uniformity |
|---|---|
| Example 1 | 101.8%, RSD 1.2% |
| Example 4 | 101.2%, RSD 5.4% |

The RSD limit for the sample of the invention from Example 1 is significantly less than the 5.0 percent that is acceptable by the FDA and the 5.4 percent of Comparative Example 4. Thus, as illustrated in Table 7, the granulated memantine compositions of the invention prepared by wet granulation fall within acceptable RSD limits, while those prepared by direct compression fall outside acceptable RSD limits, i.e., suffer from a lack of uniformity.

Further, as illustrated by the data presented above, the granulated memantine compositions of the invention, preferably manufactured by wet granulation, have excellent physical and chemical characteristics. In addition, in view of physical processing considerations, the preferred method of manufacture for memantine tablets was found to be wet granulation. When using wet granulation, there is no need to produce harder tablets in order to avoid sticking, and there is no need to increase blending time to reach good blend uniformity.

### Example 5: Memantine Composition Prepared by Dry Granulation

Memantine hydrochloride (5 mg/tablet), lactose monohydrate (35 mg/tablet), povidone (1 mg/tablet), croscarmellose sodium (7 mg/tablet), and magnesium stearate (0.5 mg/tablet) **[Part I]** are blended to form a mixture. The mixture is then compacted or slugged, and then milled to form granules. The resulting granules are then blended with croscarmellose sodium (2 mg/tablet), microcrystalline cellulose (32.5 mg/tablet), and colloidal silicon dioxide (1 mg/tablet) **[Part II]** and magnesium stearate (1 mg/tablet) **[Part III]** to form the composition. The total blending time is 30 minutes. The ingredients are summarized in Table 8.

**Table 8**

| Ingredient | (mg/tablet) |
|---|---|
| **Part I:** | |
| Memantine HCl | 5 |
| Lactose Monohydrate | 35 |
| Microcrystalline Cellulose | 35 |
| Povidone | 1 |
| Croscarmellose Sodium | 7 |
| Magnesium Stearate | 0.5 |
| | |

| **Part II** | |
|---|---|
| Croscarmellose Sodium | 2 |
| Microcrystalline Cellulose | 32.5 |
| Colloidal Silicon Dioxide | 1 |
| | |

| **Part III** | |
|---|---|
| Magnesium Stearate | 1 |
| OPADRY® | 4 |
| | |
| Total | 124 |

### Example 6: Memantine Composition Prepared by Dry Granulation

Memantine hydrochloride (10 mg/tablet), lactose monohydrate (50 mg/tablet), microcrystalline cellulose (100 mg/tablet), croscarmellose sodium (8 mg/tablet), and magnesium stearate (1 mg/tablet) **[Part I]** are blended to form a mixture. The mixture is then compacted or slugged, and then milled to form granules. The resulting granules are then blended with croscarmellose sodium (4 mg/tablet), lactose monohydrate (50 mg/tablet), and talc (5 mg/tablet) **[Part II]** and magnesium stearate (2 mg/tablet) **[Part III]** to form the composition. The total blending time is 30 minutes. The ingredients are summarized in Table 9.

**Table 9:**

| Ingredient | (mg/tablet) |
|---|---|
| **Part I** | |
| Memantine HCl | 10 |
| Lactose Monohydrate | 50 |
| Microcrystalline Cellulose | 100 |
| Croscarmellose Sodium | 8 |
| Magnesium Stearate | 1 |
| | |

| **Part II** | |
|---|---|
| Croscarmellose Sodium | 4 |
| Lactose Monohydrate | 60 |
| Talc | 5 |
| | |

| **Part III** | |
|---|---|
| Magnesium Stearate | 2 |
| OPADRY® | 8 |
| | |
| Total | 248 |

all such modifications and embodiments as falling within the true spirit and scope of the present invention.

## Claims

1. A composition comprising granulated memantine or a pharmaceutically acceptable salt thereof.

2. The composition of claim 1, comprising wet granulated memantine or a pharmaceutically acceptable salt thereof.

3. The composition of claim 1 or claim 2, wherein about 95 percent of the memantine is released within about 60 minutes, preferably 45 minutes, more preferably 30 minutes, most preferably 15 minutes, of placement of the composition in 900 ml of 0.1 N HCl at 37°C in a basket apparatus rotating at 100 rpm.

4. The composition of any one of the preceding claims, further comprising at least one pharmaceutically acceptable excipient.

5. The composition of claim 4, wherein the pharmaceutically acceptable excipient is selected from the group consisting of diluents, binders, disintegrants, glidants, and lubricants.

6. The composition of claim 5, wherein the diluent is at least one of lactose monohydrate or microcrystalline cellulose.

7. The composition of claim 5 or claim 6, wherein the diluent is present in an amount of about 40 percent to about 95 percent by weight of the composition.

8. The composition of claim 5, wherein the binder is povidone

9. The composition of claim 5 or claim 8, wherein the binder is present in an amount of about 0.5 percent to about 10 percent by weight of the composition.

10. The composition of claim 5, wherein the disintegrant is croscarmellose sodium.

11. The composition of claim 5 or claim 10, wherein the disintegrant is present in an amount of about 3 percent to about 10 percent by weight of the composition.

12. The composition of claim 5, wherein the glidant is at least one of colloidal silicon dioxide or talc.

13. The composition of claim 5 or claim 12, wherein the glidant is present in an amount of about 0.5 percent to about 3 percent by weight of the composition.

14. The composition of claim 5, wherein the lubricant comprises at least one of magnesium stearate, sodium stearyl fumarate, and talc.

15. The composition of claim 5 or claim 14, wherein the lubricant is present in an amount of about 0.5 percent to about 2 percent by weight of the composition.

16. The composition of any one of claims 4 to 15, comprising a granulated mixture of memantine or a pharmaceutically acceptable salt thereof and all or part of each pharmaceutically acceptable excipient.

17. The composition of any one of the preceding claims, wherein the granulated memantine is granulated memantine hydrochloride.

18. The composition of any one of the preceding claims, wherein the memantine or pharmaceutically acceptable salt thereof is present in an amount from about 2 percent to about 6 percent, preferably about 4 percent, by weight of the composition.

19. A pharmaceutical dosage form, comprising a composition as defined in any one of the preceding claims.

20. The pharmaceutical dosage form of claim 19, wherein the dosage form has a uniformity of content for a plurality of the dosage forms, such that 10 individually sampled dosage forms have a mean assay of between 90 to 110 percent of a target value and a relative standard deviation of not more than 5.0 percent.

21. The pharmaceutical dosage form of claim 19 or claim 20 in solid form.

22. The pharmaceutical dosage form of claim 21, wherein the solid oral dosage form is a tablet, a capsule, or a sachet of granules.

23. The pharmaceutical dosage form of claim 22, wherein the solid oral dosage form is a film-coated tablet.

24. The dosage form of any one of claims 19 to 23, comprising memantine HCl present in an amount of about 5 mg, lactose monohydrate present in an amount of about 70 mg, povidone present in an amount of about 1 mg, croscarmellose sodium present in an amount of about 9 mg, microcrystalline cellulose present in an amount of about 32.5 mg, colloidal silicon dioxide present in an amount of about 1 mg, and magnesium stearate present in an amount of about 1.5 mg.

25. The dosage form of any one of claims 19 to 23, comprising memantine HCl present in an amount of about 10 mg, lactose monohydrate present in an amount of about 140 mg, povidone present in an amount of about 2 mg, croscarmellose sodium present in an amount of about 18 mg, microcrystalline cellulose present in an amount of about 65 mg, colloidal silicon dioxide present in an amount of about 2 mg, and magnesium stearate present in an amount of about 3 mg.

26. A wet granulation process for preparing a memantine composition, comprising granulating memantine or a pharmaceutically acceptable salt thereof with a liquid to form a granulate, and forming a composition from the granulate.

27. The process of claim 26, wherein the liquid is water.

28. The process of claim 26 or claim 27, further comprising drying and milling the granulate prior to forming the composition.

29. The process of any one of claims 26 to 28, further comprising blending the memantine with at least one pharmaceutically acceptable excipient prior to granulating with the liquid.

30. The process of any one of claims 26 to 29, further comprising blending the granulate with at least one additional pharmaceutically acceptable excipient to form the composition.

31. The process of claim 29 or claim 30, wherein the at least one pharmaceutically acceptable excipient and the additional pharmaceutically acceptable excipient are selected independently from the group consisting of diluents, glidants, disintegrants, and lubricants.

32. The process of any one of claims 26 to 31 for preparing a composition as defined in any one of claims 1 to 18.

33. A dry granulation process for preparing a memantine composition, comprising compacting or slugging memantine or a pharmaceutically acceptable salt thereof, milling the resultant compact or slugs to form granules, and forming a composition from the granules.

34. The process of claim 33, further comprising mixing the memantine and at least one pharmaceutically acceptable excipient to form a mixture, prior to compaction or slugging.

35. The process of claims 33 or claim 34, wherein the composition is formed from the granules by blending the granulate with at least one additional pharmaceutically acceptable excipient.

36. The process of claim 35, wherein the additional pharmaceutically acceptable excipient is selected from the group consisting of diluents, glidants, disintegrants, and lubricants.

37. A pharmaceutical composition comprising memantine or a pharmaceutically acceptable salt thereof obtainable by a process as defined in any one of claims 26 to 36.

38. A composition as defined in any one of claims 1 to 18 and 37 or a dosage form as defined in any one of claims 19 to 25 for use in therapy.

39. A composition as defined in any one of claims 1 to 18 and 37 or a dosage form as defined in any one of claims 19 to 25 for use in treating Alzheimer's disease.
